Europäisches Patentamt

**European Patent Office**

**Office européen des brevets**

(11) Veröffentlichungsnummer: **0 204 343**
**B1**

(12) **EUROPÄISCHE PATENTSCHRIFT**

(45) Veröffentlichungstag der Patentschrift:
**03.05.89**

(51) Int. Cl.⁴: **G 01 N 1/10,** G 01 N 35/00,
G 01 N 35/06

(21) Anmeldenummer: **86107720.4**

(22) Anmeldetag: **06.06.86**

(54) **Fraktionshandhabungseinrichtung.**

(30) Priorität: **07.06.85 DE 3520438**

(43) Veröffentlichungstag der Anmeldung:
**10.12.86 Patentblatt 86/50**

(45) Bekanntmachung des Hinweises auf die Patenterteilung:
**03.05.89 Patentblatt 89/18**

(84) Benannte Vertragsstaaten:
**BE CH DE FR GB IT LI NL**

(56) Entgegenhaltungen:
**CH-A- 531 173**
**DE-A- 3 015 051**
**US-A- 4 422 151**

(73) Patentinhaber: **Erweka Apparatebau GmbH,**
**Ottostrasse 20-22, D-6056 Heusenstamm (DE)**

(72) Erfinder: **Schneider, Ortwin, Arheilgerstrasse 39,**
**D-6108 Weiterstadt (DE)**
Erfinder: **Georgitsis, Nikolaos, Kolpingstrasse 34,**
**D-6056 Heusenstamm (DE)**

(74) Vertreter: **Patentanwälte Kirschner & Grosse,**
**Forstenrieder Allee 59, D-8000 München 71 (DE)**

## Beschreibung

Die Erfindung betrifft eine Fraktionshandhabungseinrichtung nach dem Oberbegriff des Hauptanspruches.

Fraktionssammler dienen dazu, in regelmäßigen Abständen Proben aus Meßbehältern zu entnehmen und eine den Proben entsprechende Menge an Ergänzungsflüssigkeit in die Meßbehälter zurückzufördern. Wenn diese Einrichtungen in der pharmazeutischen Industrie eingesetzt werden, dienen sie zur Entnahme von Fraktionen von Arzneimittellösungen, deren Langzeitverhalten bei Körpertemperatur (37 °C) bestimmt werden soll. Bei diesen Messungen ist es wichtig, daß eine bestimmte Messung nicht durch die vorangehende Messung beeinflußt wird, d.h., daß die Leitungen, durch die die Meßproben fließen, zwischen den einzelnen Probenentnahmen gesäubert werden müssen. Außerdem muß sichergestellt sein, daß genau die gleiche Menge an Ergänzungsflüssigkeit in die Meßbehälter zurückgeführt wird, die vorher als Probe entnommen wurde. Dazu wird bei einem früheren Fraktionssammler Ergänzungsflüssigkeit über die gleiche Pumpe in die Meßbehälter gefördert, über die auch die Entnahme der Probe erfolgt. Zum Einleiten der Ergänzungsflüssigkeit in den Pumpenkreislauf ist ein Ventil erforderlich, um den Leitungszug für die Proben und den Leitungszug für die Ergänzungsflüssigkeit zu verwirklichen. Die frühere Einrichtung hat den Nachteil, daß nicht die gesamte Meßleitung mit der Ergänzungsflüssigkeit gespült wird, weil die Leitungen für die Ergänzungsflüssigkeit von dem zugehörigen Vorratsbehälter zu dem Ventil separat von den Leitungen von dem Ventil zu den Reagenzgläsern für die Meßproben angeordnet sind. Zwischen den Ventilen und den Meßbehältern treten die Flüssigkeitsproben durch dieselben Leitungen wie die Ergänzungsflüssigkeit hindurch, wobei die beiden Flüssigkeiten selbstverständlich in entgegengesetzter Richtung fließen.

Aus der DE-AS 19 14 701 ist ein Fraktionssammler bekannt, der eine in einem Vorratsbehälter vorhandene Flüssigkeit über eine bewegliche Zuleitung an verschiedene Behälter verteilt. In der Leitung ist eine Pumpe vorgesehen und über ein Magnetventil, das an einem längs einer Schiene über die Behälter positionierbaren Wagen angeordnet ist, wird den Behältern die Flüssigkeit tropfenweise zugeführt. Eine Entnahme von Proben aus einem Meßbehälter und damit ein Zuführen der Proben in Probenbehälter ist in dieser DE-AS nicht beschrieben.

Die DE-AS 21 59 430 offenbart eine Vorrichtung für chemische Analysen, bei der mittels einer Sonde aus einem Meßbehälter eine Probe angesaugt wird. Anschließend wird die Sonde zu einem Probenbehälter transportiert, in den die Probe abgelassen wird. Zusätzlich kann aus einem Vorratsbehälter eine weitere Flüssigkeit über die Sonde in den Probenbehälter abgelassen werden. Eine Ergänzung der dem Meßbehälter entnommenen Flüssigkeit ist bei dieser bekannten Vorrichtung nicht vorgesehen.

Aus der US-PS 3 912 456 ist ebenfalls eine Vorrichtung für chemische Analysen bekannt, bei der eine Sonde an einem Ansaugarm und Röhrchen an einem Verteilarm vorgesehen sind. Über die Sonde wird eine Probe aus einem Meßbehälter angesaugt und diese Probe wird mittels einer Fördereinrichtung zum Verteilarm übertragen und dort mittels eines Röhrchens in einen Probenbehälter eingefüllt. Anschließend werden die Sonde und die Röhrchen sowie die Verbindungsleitung zwischen beiden mit einer Flüssigkeit gespült. Bei dieser bekannten Vorrichtung ist ebenfalls keine Ergänzung der dem Meßbehälter entnommenen Flüssigkeit vorgesehen und außerdem ist der Spülvorgang ausdrücklich vorgesehen.

Der Erfindung liegt demgegenüber die Aufgabe zugrunde, eine Fraktionshandhabungseinrichtung zu schaffen, bei der auf einfache Weise die Flüssigkeitsmenge der jeweils aus einem Meßbehälter entnommenen Probe durch eine entsprechende Ergänzungsflüssigkeitsmenge ersetzt wird und bei der ein Spülen der Leitungen, der Sonden und der Röhrchen mit einer weiteren Flüssigkeit nicht erforderlich ist, wobei die Einrichtung auch auf einfache Weise zur Durchführung zusätzlicher Funktionen, beispielsweise das Verdünnen der Flüssigkeitsproben und das Fördern der verdünnten Flüssigkeitsproben zu einer Meßeinheit, weiter ausbaufähig sein soll.

Die erfindungsgemäße Fraktionshandhabungseinrichtung ist dazu in der im Hauptanspruch angegebenen Weise gekennzeichnet. Bei der erfindungsgemäßen Einrichtung wird die gesamte Meßleitung mit Ergänzungsflüssigkeit zwischen zwei Probenentnahmen gespült. Der zusätzliche Bauaufwand ist gering und besteht im wesentlichen nur aus der Einrichtung, die erforderlich ist, um die Röhrchen in den Vorratsbehälter zu führen.

Eine einfache Ausführungsform für die Relativbewegung zwischen Röhrchen und dem Vorratsbehälter ist in Anspruch 2 gekennzeichnet. Da ein Motor und eine entsprechende Einrichtung für die Auf- und Abbewegung der Röhrchen auch dazu vorhanden sein muß, daß die Röhrchen in die Probenbehälter (Reagenzgläser) hineingefahren und aus diesen herausbewegt werden können, ist bei dieser Lösung nur die Einrichtung zum Verfahren des Vorratsbehälters zusätzlich erforderlich.

Die vorteilhafte Ausgestaltung der Erfindung nach Anspruch 3 hat den Vorteil, daß zusätzliche Funktionen, nämlich das Verdünnen der Meßproben in den Reagenzgläsern und das Zuführen der verdünnten Meßproben zu einer Meßeinheit, mit geringem zusätzlichem Konstruktionsaufwand verwirklicht werden können.

Die Ausgestaltung der Erfindung nach Anspruch 4 hat den Vorteil, daß die konstruktiven Maßnahmen zur Verwirklichung der zusätzlichen Funktionen lediglich darin bestehen, daß ein weiterer Vorratsbehälter für die Verdünnungsflüssigkeit vorzusehen ist, während die Steuerung der Bewegung der Röhrchen und das Verfahren der Vorratsbehälter ebenso einfach ist wie bei dem ersten Ausführungsbeispiel.

Die Ausgestaltung des ersten und des zweiten Ausführungsbeispieles der Erfindung nach Anspruch 5 hat den Vorteil, daß der oder die Vorratsbehälter in dem engen Raum zwischen den Röhrchen und den Probenbehältern (Reagenzgläsern) angeordnet werden kann/können und dennoch immer genügend Ergänzungsflüssigkeit im betreffenden Vorratsbehälter enthalten ist, um funktionsfähig zu sein.

Die Ausgestaltung der Erfindung nach Anspruch 6 hat den Vorteil, daß der oder die Vorratsbehälter automatisch auf einfache Weise nachgefüllt werden, so daß der Füllstand immer auf einem genügend hohen Niveau ist.

Ausführungsbeispiele der Erfindung werden nun anhand der beiliegenden Zeichnungen beschrieben. Es zeigen:

Fig. 1 eine z.T. schematische, z.T. geschnittene Seitenansicht eines ersten Ausführungsbeispiels einer Fraktionshandhabungseinrichtung;

Fig. 2 eine weitere Seitenansicht der in Fig. 1 gezeigten Fraktionshandhabungseinrichtung;

Fig. 3 eine z.T. schematische, z.T. geschnittene Seitenansicht eines zweiten Ausführungsbeispiels einer Fraktionshandhabungseinrichtung; und

Fig. 4 eine weitere Seitenansicht der in Fig. 3 gezeigten Fraktionshandhabungseinrichtung.

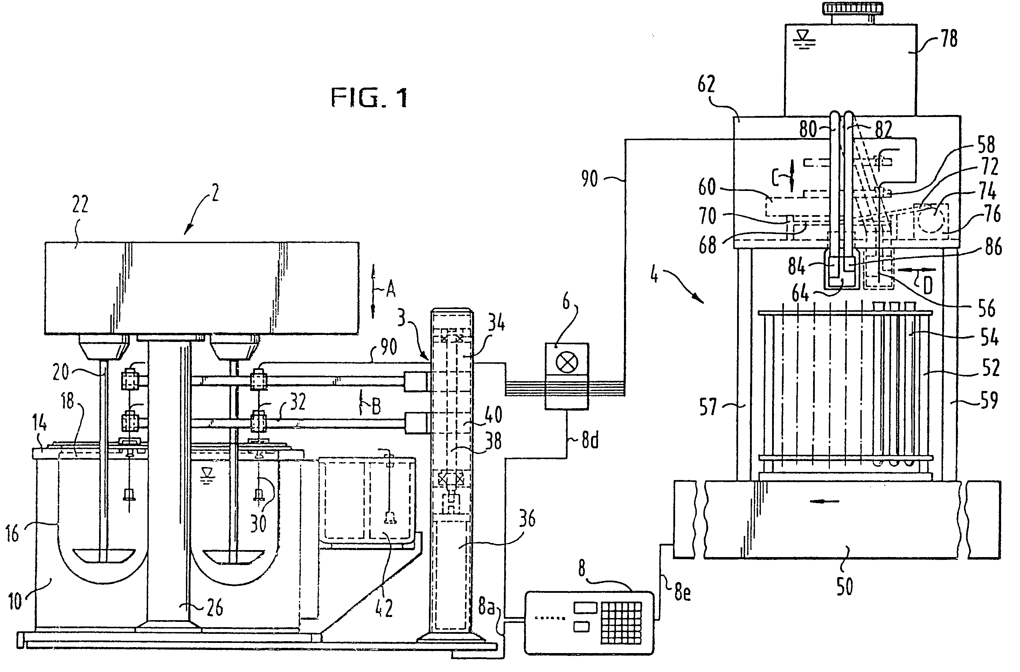

Gemäß Fig. 1 besteht die Fraktionshandhabungseinrichtung aus einer Meßbehältereinheit 2, einer Probenentnahmeeinheit 3, einer Probensammeleinheit 4, einer sechsteiligen Schlauchpumpe 6 (im Falle von sechs Leitungen von sechs Reaktions- oder Meßbehältern) und einem Steuergerät 8. Das Steuergerät 8 ist mit der Meßbehältereinheit 2 und der Probenentnahmeeinheit 3 durch eine Leitung 8a, mit der Probensammeleinheit 4 mit einer Leitung 8e und mit der Schlauchpumpe 6 durch eine Leitung 8d verbunden, um die erforderlichen Steuersignale an die jeweiligen Einheiten abgeben zu können.

Ein Wasserbehälter 10 mit einem Thermostat 12 ist in der Meßbehältereinheit 2 vorgesehen. In die Deckplatte 14 des Wasserbehälters 10 sind sechs Meßbehälter 16 eingehängt und mit Deckeln 18 verschlossen. In die Meßbehälter 16 ragen Rührer 20 hinein, die in einem Gehäuse 22 gelagert sind, das auch den Antrieb für die Rührer enthält. Das Gehäuse 22 ist mit zwei Teleskopsäulen 24, 26 auf einer Grundplatte 27 angeordnet und kann auf- und abbewegt werden, wie durch den Doppelpfeil A in Fig. 1 angedeutet ist.

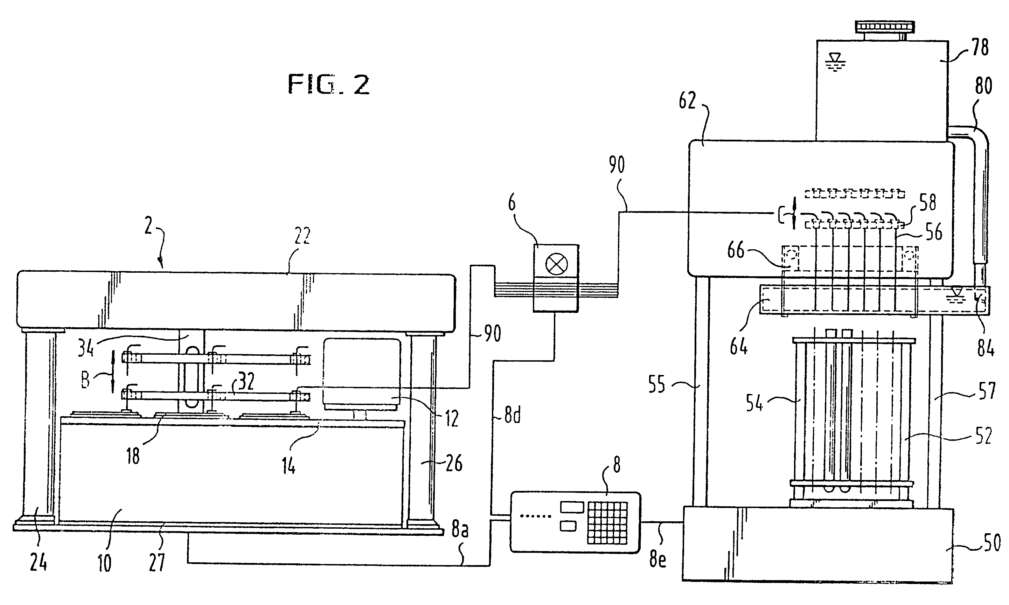

Um aus den Meßbehältern 16 Proben zu entnehmen, sind Sonden 30 auf einem Rahmen 32 angeordnet, der an einer Säule 34 auf- und abbewegbar ist, wie durch den Doppelpfeil B in Fig. 1 dargestellt ist. Der Rahmen 32 ist in den Fig. 1 und 2 sowohl in seiner unteren als auch in seiner oberen Endlage dargestellt. Die Bewegung des Rahmens 32 wird durch einen Motor 36 bewirkt, der eine Spindel 38 antreibt, auf der der Rahmen mit einer Mutter 40 gelagert ist.

In der Probenentnahmeeinheit 2 ist schließlich noch ein Verwurfbehälter 42 neben dem Wasserbehälter 10 vorgesehen, in den die Sonden 30 abgelegt werden können.

Die Probensammeleinheit 4 hat ein Grundgehäuse 50, in dem eine Fördereinrichtung für ein Gestell 52 mit Reagenzgläsern 54 (Probenbehälter) angeordnet ist, in die die flüssigen Proben eingefüllt werden sollen. In dem gezeigten Ausführungsbeispiel sind mehrere Reihen von je sechs Reagenzgläsern in dem Gestell 52 angeordnet, und das Gestell 52 wird nach jeder Probenentnahme um eine Reihe weiterbewegt. Über dem Grundgehäuse 50 ist auf Säulen 55, 57, 59 ein weiteres Gehäuse 62 angeordnet, in dem über den Reagenzgläsern 54 ein Röhrchen 56 (im vorliegenden Beispiel sechs Röhrchen) liegen, die über sechs, schematisch dargestellte Schlauchleitungen 90 mit den Sonden 30 verbunden sind und oberhalb der Reagenzgläser liegen. Die Röhrchen 56 sind in einer Halterung 58 befestigt, die durch einen Motor 60 auf- und abbewegbar ist, wie durch den Doppelpfeil C in Fig. 1 dargestellt ist. In den Fig. 1 und 2 ist die Halterung 58 sowohl in der unteren als auch in der oberen Endlage dargestellt. Auf der Unterseite des Gehäuses 62, in dem die Röhrchen 56 gelagert sind, ist eine Wanne 64 angeordnet, die zwischen einer Stellung unterhalb der Röhrchen und einer Stellung, in der der Weg zwischen den Röhrchen 56 und den Reagenzgläsern 54 freigegeben ist, hin- und herbewegt werden kann, wie durch den Pfeil D in Fig. 1 angedeutet ist. Die Wanne 64 ist an einem Rahmen 66 aufgehängt, der seinerseits auf zwei Führungsschienen 68 gelagert ist, die an dem Gehäuse 62 an Haltern 70 befestigt sind. Der Rahmen 66 ist über eine Kurbel 72 mit dem Antriebsrad 74 eines Motors 76 verbunden, der die Hin- und Herbewegung der Wanne 64 bewirkt.

Da für die Wanne 64 zwischen den Röhrchen 56 und den Reagenzgläsern 54 nur wenig Platz zur Verfügung steht, ist ihr Volumen entsprechend gering. Daher muß dafür gesorgt werden, daß die Wanne 64 nachgefüllt wird, wenn aus ihr Flüssigkeit entnommen worden ist. Dazu ist ein Nachfüllbehälter 78 auf dem Gehäuse 62 vorgesehen, von dem zwei Schläuche 80, 82 in die Wanne 64 führen. Die Schläuche 80, 82 sind an zwei Kunststoffrohren 84, 86 befestigt, die ihrerseits an der Wanne 64 befestigt sind und die unterschiedlich tief in die Wanne hineinragen. Wenn der Flüssigkeitspegel in der Wanne 64 unter das Niveau des kürzeren Rohres 86 absinkt, tritt Luft in den Behälter 78 ein, und eine entsprechende Menge Flüssigkeit fließt über den Schlauch 80 in die Wanne, bis beide Rohre 84, 86 mit ihren Enden wieder unterhalb des Flüssigkeitspegels liegen. Auf diese Weise wird die Befüllung der Wanne 64 sichergestellt.

Die vorstehend beschriebene Fraktionshandhabungseinrichtung arbeitet wie folgt. Unter der Steuerung des Steuergeräts 8 wird das Gestell 52 mit den Reagenzgläsern 54 in eine Position gebracht, in der die Reagenzgläser 54 jeweils unter den Röhrchen 56 stehen. Die Wanne 64 befindet sich dabei in der Position, in der sie den Weg zwischen den Röhrchen 56 und den Reagenzgläsern 54 freigibt. Die Sonden 30 werden in die Flüssigkeit in den Meßbehältern 16 abgesenkt. Dann wird die Pumpe 6 angesteuert, so daß Flüssigkeit aus den Meßbehältern 16 über die Sonden 30 abgezogen, von der Pumpe 6 durch die Leitungen 90 (schematisch dargestellt) zu den Röhrchen 56 gepumpt und von dort durch die Röhrchen 56 in die Reagenzgläser 54 abgegeben wird. Die Menge der Flüssigkeit, die von den Meßbehältern 16 entnommen wird, wird durch die Zahl der Umdrehungen des Motors der Pumpe 6 festgelegt. Sobald die Pumpe die erforderliche Zahl von Umdrehungen ausgeführt hat, stoppt die Pumpe, und die Sonden 30 werden aus den Meßbehältern 16 herausgefahren. Danach arbeitet die Pumpe erneut, um die Flüssigkeit in derselben Strömungsrichtung wie vorher weiterzupumpen, so daß die Leitungen 90 in die Reagenzgläser 94 entleert werden.

Nach einer solchen Probenentnahme muß genau die gleiche Flüssigkeitsmenge, die vorher als Probe aus den Meßbehältern 16 entnommen wurde, durch Ergänzungsflüssigkeit ersetzt werden. Daher werden die Röhrchen nach Beendigung der Probenentnahme nach oben gefahren, und die Wanne 64 wird in die Stellung unterhalb der Röhrchen gefahren. Dann werden die Röhrchen wieder abgesenkt, so daß sie in die in der Wanne 64 befindliche Ergänzungsflüssigkeit eintauchen. Danach wird die Pumpe 6 von dem Steuergerät 8 derart angesteuert, daß sie die gleiche Anzahl von Umdrehungen, die sie bei der Probenentnahme vorwärts gelaufen ist, rückwärtsläuft, so daß die den Proben entsprechende Menge an Ergänzungsflüssigkeit in die Meßbehälter 16 zurückgepumpt wird. Bei der Nachfüllung von Ergänzungsflüssigkeit werden die Leitungen 90 ebenfalls nach dem Ziehen der richtigen Menge von Ergänzungsflüssigkeit aus der Wanne 64 geleert. Zu diesem Zweck leert die Pumpe 6 die Leitungen 90 in die Meßbehälter 16. Bei dem Zurückpumpen von Ergänzungsflüssigkeit wird die gesamte Leitung von den Röhrchen 56 bis zu den Sonden 30 mit Ergänzungsflüssigkeit gespült, so daß bei der nachfolgenden Probenentnahme keine Fehler aufgrund von Verunreinigungen aus der vorangehenden Probenentnahme entstehen können. Nach dem Zurückführen der Ergänzungsflüssigkeit werden die Röhrchen 56 wieder nach oben gefahren und die Wanne 64 wird wieder in die Ruhestellung gebracht. Sodann ist die Fraktionshandhabungseinrichtung für die nächste Probenentnahme vorbereitet, die nach einer durch das Steuergerät 8 einstellbaren Zeit stattfindet.

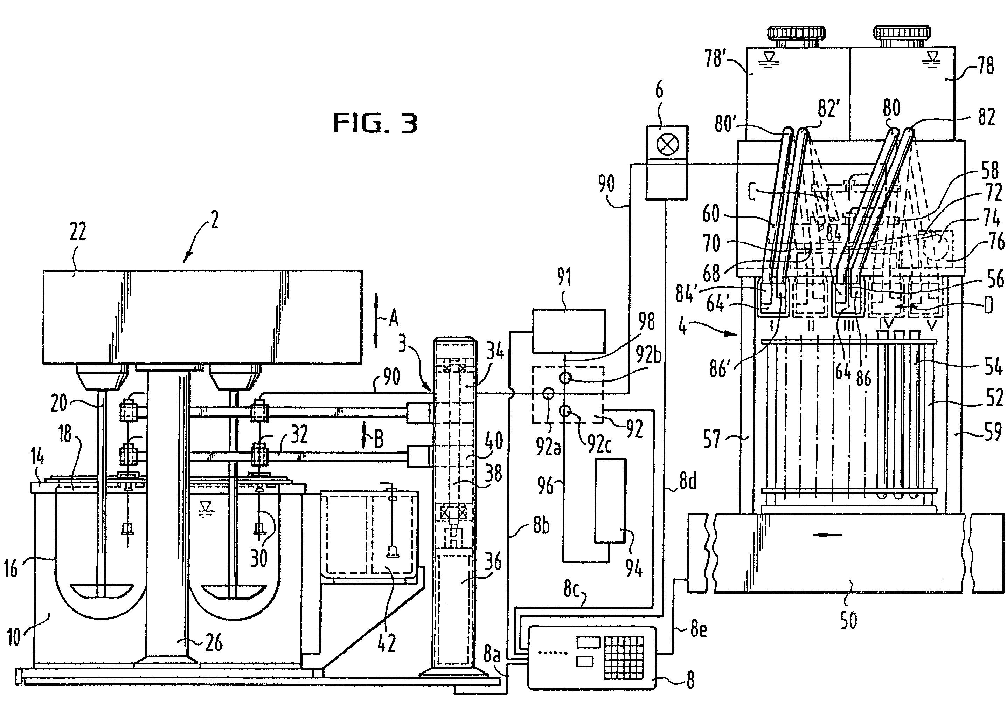

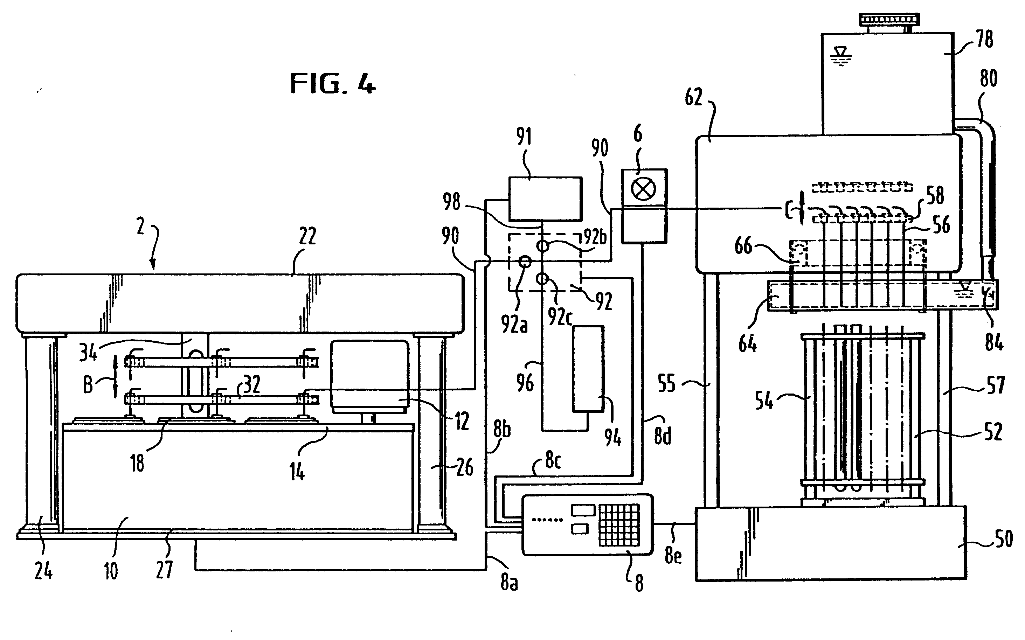

Bei dem zweiten Ausführungsbeispiel der Erfindung (Fig. 3 und 4) werden dieselben Bezugsziffern für die gleichen Merkmale wie bei dem oben beschriebenen Ausführungsbeispiel verwendet. Das zweite Ausführungsbeispiel ist mit dem ersten Ausführungsbeispiel in mehrfacher Hinsicht identisch, und die identischen Teile werden nicht erneut beschrieben, sondern nur die konstruktiven und funktionellen Abwandlungen des zweiten Ausführungsbeispieles gegenüber dem ersten. Das zweite Ausführungsbeispiel weist zusätzlich zu dem ersten Ausführungsbeispiel eine zusätzliche Vorratseinrichtung 64', 78' für die Verdünnungsflüssigkeit, eine Ventileinheit 92 mit drei Ventilen 92a, 92b und 92c, einen Flüssigkeitsbehälter 94, eine Flüssigkeitsleitung 96, die die Ventileinheit 92 mit dem Flüssigkeitsbehälter 94 verbindet, und eine Flüssigkeitsleitung 98 auf, die die Ventileinheit 92 mit einem Meßgerät 91 verbindet. Die Leitungen 96 und 98 sind (wie die Leitung 90) nur schematisch dargestellt und bestehen bei dem gezeigten Ausführungsbeispiel aus sechs Einzelleitungen, wenn sechs Proben gleichzeitig verarbeitet werden. Der Flüssigkeitsbehälter 94 besteht aus sechs Teilbehältern, wenn sechs Proben gleichzeitig verarbeitet werden. Eine zusätzliche Steuerleitung 8b verbindet das Steuergerät 8 mit dem Meßgerät 91, und eine weitere Steuerleitung 8c verbindet das Steuergerät 8 mit der Ventileinheit 92, um diese Einheiten jeweils zu steuern. Die Ventileinheit 92 liegt in der Leitung 90 zwischen der Pumpe 6 und den Sonden 30, um die im folgenden beschriebenen Funktionen durchführen zu können.

Die Vorratseinrichtung für Verdünnungsflüssigkeit ist ebenso aufgebaut wie die oben beschriebene Vorratseinrichtung für Ergänzungsflüssigkeit. Im einzelnen weist die Verdünnungsflüssigkeit-Vorratseinrichtung eine Wanne 64' auf, die in dem Raum zwischen den Röhrchen 56 (wenn diese sich in ihrer oberen Endposition befinden) und den Reagenzgläsern 54 bewegbar ist. Die Wanne 64' ist mit einem Nachfüllbehälter 78' verbunden, der auf dem Gehäuse 62 neben dem Ergänzungsflüssigkeits-Nachfüllbehälter 78 angeordnet ist. Zwei Schläuche 80', 82' führen von dem Boden des Nachfüllbehälters 78' zu dem Trog 64'. Die Schläuche 80', 82' sind an zwei Kunststoffrohren 84', 86' befestigt, die ihrerseits an der Wanne 64' befestigt sind, und die unterschiedlich tief in die Wanne 64' hineinragen. Durch diese Anordnung wird der Flüssigkeitspegel in der Wanne 64' für die Verdünnungsflüssigkeit so geregelt wie der Flüssigkeitspegel in der Wanne 64 für die Ergänzungsflüssigkeit.

Eine weitere Modifikation des zweiten Ausführungsbeispiels ist in der Antriebseinrichtung für die Auf- und Abbewegung der Röhrchen 56 und in der Antriebseinrichtung zur Bewegung der Wannen 64 und 64' gegeben. Die Antriebseinrichtung zur Bewegung der Röhrchen 56 in Richtung des Pfeiles C sind so ausgeführt, daß sie die Röhrchen 56 in eine erste Position, in der die unteren Enden der Röhrchen 56 oberhalb der Wannen 64, 64' liegen, in eine zweite Position, in der die Röhrchen 56 in die Flüssigkeit in den Wannen 64 oder 64' eingetaucht sind (je nachdem, welcher Trog gerade unterhalb der Röhrchen 56 liegt), und eine dritte Position bewegt werden können, in der die Röhrchen 56 in die Flüssigkeit in den Reagenzgläsern 54 eingetaucht sind. Um die Flüssigkeit in den Reagenzgläsern 54 während der Verdünnung der Proben zu durchmischen oder aufzurühren, werden die Röhrchen 56 periodisch auf- und abbewegt, wobei ihre unteren Enden immer unterhalb des Flüssigkeitspegels in den Reagenzgläsern 54 bleiben. Um eine bessere Durchmischung der Flüssigkeit während der Verdünnung zu erreichen, haben die Röhrchen 56 bei dem zweiten Ausführungsbeispiel an ihren unteren Enden eine Mischeinrichtung, beispielsweise Paddeln (nicht gezeigt). Die Antriebseinrichtung zur Bewegung der Wannen 64 und 64' ist insoweit gegenüber der entsprechenden Antriebseinrichtung in dem ersten Ausführungsbeispiel abgewandelt, daß beide Wannen 64, 64' gleichzeitig bewegt werden, wobei die Wanne 64 für Ergänzungsflüssigkeit unterhalb der Röhrchen 56, oder die Wanne 64' für Verdünnungsflüssigkeit unterhalb der Röhrchen 56 zu liegen kommt oder beide Wannen 64, 64' auf gegenüberliegenden Seiten der Röhrchen 56 zu liegen kommen. Fig. 3 zeigt fünf Positionen, die von der Wanne 64 und/oder der Wanne 64' eingenommen werden können. Wenn die Wanne 64' sich in Position I befindet, ist die Wanne 64 in Position III, d.h. unterhalb der Röhrchen 56. Wenn die Wanne 64' in der Position II ist, ist die Wanne 64 in Position IV, so daß die Röhrchen 56 sich frei in die Reagenzgläser 54 nach unten bewegen können. Wenn die Wanne 64' in Position III ist, ist die Wanne 64 in der Position IV, d.h., daß die Wanne 64' unterhalb der Röhrchen 56 liegt. Diese Bewegung der Wannen kann durch dieselbe Art von Motor und Antriebsgestänge verwirklicht werden, wie im Zusammenhang mit dem ersten Ausführungsbeispiel erläutert wurde.

Die Funktionsweise des zweiten Ausführungsbeispiels ist wie folgt. Während der Probenentnahme aus den Meßbehältern 16 und während der Nachfüllung von Ergänzungsflüssigkeit in die Meßbehälter 16 ist das Ventil 92a geöffnet, und die Ventile 92b und c sind geschlossen. Die Probenentnahme und die Nachfüllung von Ergänzungsflüssigkeit wird so vorgenommen, wie es oben im Zusammenhang mit dem ersten Ausführungsbeispiel beschrieben wurde. Nachdem die flüssigen Proben in die Reagenzgläser 54 eingefüllt worden sind, sollen die Proben nun verdünnt werden. Zu diesem Zweck wird die Wanne 64' mit der Verdünnungsflüssigkeit in die Position III bewegt, die Röhrchen 56 werden in die Verdünnungsflüssigkeit eingetaucht, die Ventile 92a und 92b werden geschlossen, und das Ventil 92c wird geöffnet. Die Pumpe 6 wird dann im Uhrzeigersinn betrieben, um die Verdünnungsflüssigkeit von der Wanne 64' zu den Teilbehältern in dem Behälter 94 zu fördern. Nachdem die richtige Menge von Verdünnungsflüssigkeit von der Pumpe 6 angesaugt wurde, werden die Röhrchen 56 aus der Wanne 64' herausbewegt, und die Wanne 64' wird in die Position II gebracht. Die Röhrchen 56 werden in die Reagenzgläser 54 hineinbewegt, und die Pumpe 6 wird im Gegenuhrzeigersinn betätigt, um die Verdünnungsflüssigkeit aus dem Behälter 94 abzuziehen und diese Verdünnungsflüssigkeit in die Reagenzgläser 54 abzugeben. Während des Verdünnungsvorganges werden die Röhrchen 56 in den Reagenzgläsern 54 auf- und abbewegt, um die darin enthaltene Flüssigkeit zu durchmischen.

Als nächstes sollen die in den Reagenzgläsern 54 enthaltenen, verdünnten Proben dem Meßgerät 91 (Detektor oder Fotometer) zugeführt werden. Dazu werden die Ventile 92a und 92c geschlossen, und das Ventil 92b wird geöffnet. Die Pumpe 6 wird im Uhrzeigersinn betrieben, während die Röhrchen 56 in die verdünnten Flüssigkeitsproben in den Reagenzgläsern 54 eingetaucht sind. Nachdem die erforderliche Menge an verdünnter Probenflüssigkeit dem Meßgerät 91 zugeführt ist, hält die Pumpe 6 an, und die Röhrchen 56 werden in ihre obere Position gebracht, wodurch die Zufuhr von verdünnten Proben an das Meßgerät abgeschlossen ist.

Wenn die Zeit zwischen zwei Probenentnahmen es zuläßt, kann die Verdünnung der Meßproben in den Reagenzgläsern und die Zufuhr der Meßproben zu dem Meßgerät nach jeder Probenentnahme durchgeführt werden. Wenn die Zeit zwischen zwei Probenentnahmen zu kurz ist, können einige oder alle Probenentnahmen — oder Ergänzungsflüssigkeitsnachfüll-Schritte als erstes ausgeführt werden, und die Verdünnung der Proben und die Zufuhr der verdünnten Proben an das Meßgerät kann danach ausgeführt werden. Im letzteren Fall wird das Gestell 52 für die Reagenzgläser 54 in seiner Ausgangsposition zurückbewegt, nachdem die Probenentnahme- und Ergänzungsflüssigkeitsnachfüll-Schritte ausgeführt worden sind, und das Gestell 52 wird dann wieder Schritt für Schritt bewegt, um die Verdünnung und die Zufuhr der Proben an das Meßgerät auszuführen.

## Patentansprüche

1. Fraktionshandhabungseinrichtung mit
— einer Probenentnahmeeinheit mit einer Vielzahl von Sonden zur Entnahme flüssiger Proben aus einer entsprechenden Anzahl von Meßbehältern einer Meßbehältereinheit,
— einer Probensammeleinheit mit einer Vielzahl von Röhrchen zur Abgabe der flüssigen Proben in Probenbehälter, die in der Probensammeleinheit unterhalb der Röhrchen angeordnet sind,
— Leitungen, die die Sonden mit den Röhrchen verbinden, und
mit einer Fördereinrichtung, die mit den Leitungen verbunden ist, um die Proben von den Sonden zu den Röhrchen und Ergänzungsflüssigkeit von einer Ergänzungsflüssigkeits-Vorratseinrichtung zu den Sonden zu fördern, dadurch gekennzeichnet, daß
— die Röhrchen (56) sowohl zur Abgabe der Proben in die Probenbehälter (54) als auch zum Ansaugen der Ergänzungsflüssigkeit aus der Vorratseinrichtung (64, 78) vorgesehen sind, wobei hierzu die Röhrchen (56) in die Vorratseinrichtung (64, 78) einführbar sind, daß
— dieselbe Fördereinrichtung (6) zum Fördern der Proben von Meßbehältern (16) in die Probenbehälter (54) bzw. zum Fördern der Ergänzungsflüssigkeit von der Vorratseinrichtung (64, 78) über die Röhrchen (56) und die Sonden (30) in die Meßbehälter (16) vorgesehen ist, und daß
— eine Steuereinrichtung (8) vorgesehen ist, um wenigstens den Betrieb der Probenentnahmeeinheit (3), der Probensammeleinheit (4) und der Fördereinrichtung (6) zu steuern.

2. Einrichtung nach Anspruch 1, dadurch gekennzeichnet, daß die Probensammeleinheit (4) eine erste Antriebseinrichtung, um die Röhrchen (56) auf und ab in eine erste Position, in der sie oberhalb dem Nachfüllbehälter (64, 78) liegen, und eine zweite Position zu bewegen, in der sie in eine Ergänzungsflüssigkeit in der Vorratseinrichtung (64, 78) eintauchen, und des weiteren eine zweite Antriebseinrichtung aufweist, um die Vorratseinrichtung (64, 78) in einem Raum zwischen den Probenbehältern (54) und den Röhrchen (56) in eine Position unterhalb der Röhrchen (56) und eine Position seitlich von den Röhrchen (56) zu bewegen.

3. Einrichtung nach Anspruch 1, gekennzeichnet durch
— eine weitere Vorratseinrichtung (64', 78'), die in der Probensammeleinheit (4) angeordnet und zur Aufnahme von Verdünnungsflüssigkeit geeignet ist, und
— eine Ventileinrichtung (92) in den Leitungen (90) zwischen der Fördereinrichtung (6) und den Sonden (30), wobei die Ventileinrichtung drei Ventile (92a, 92b, 93c) aufweist, die derart betätigbar sind, daß eine Flüssigkeitsströmung

(a) zwischen den Sonden (30) und den Röhrchen (56),

(b) zwischen den Röhrchen (56) und einem Meßgerät (91), das an eines der Ventile (92b) angeschlossen ist, und

(c) zwischen den Röhrchen (56) und einem Behälter (94), der mit einem anderen der Ventile (92c) verbunden ist, zu gestatten, wobei

— die Röhrchen (56) ferner derart angeordnet sind, daß sie in die Verdünnungsflüssigkeit in der weiteren Vorratseinrichtung (64', 78') und auch in die in den Probenbehältern (54) enthaltene Flüssigkeit eintauchbar sind, und wobei

— das Steuergerät (8) auch zur Steuerung der Ventileinrichtung (92) vorgesehen ist.

4. Einrichtung nach Anspruch 3, dadurch gekennzeichnet, daß die Probensammeleinheit (4) eine erste Antriebseinrichtung zur Auf- und Abbewegung der Röhrchen (56) in eine erste Position, in der sie oberhalb der Vorratseinrichtung (64, 78) und der weiteren Vorratseinrichtung (64', 78') liegen, in eine zweite Position, in der sie in eine Ergänzungsflüssigkeit in der Vorratseinrichtung (64, 78) oder in eine Verdünnungsflüssigkeit in der weiteren Vorratseinrichtung (64, 64') eintauchen, und eine dritte Position bewegbar sind, in der sie in die in den Probenbehältern (54) enthaltene Flüssigkeit eingetaucht sind, und daß die Probensammeleinheit (4) ferner eine zweite Antriebseinrichtung aufweist, um die Vorratseinrichtung (64, 78) und die weitere Vorratseinrichtung (64', 78') in dem Raum zwischen den Probenbehältern (54) und den Röhrchen (56) in eine Position unterhalb der Röhrchen (56) und Positionen seitlich zu den Röhrchen (56) zu bewegen.

5. Einrichtung nach einem der Ansprüche 1 bis 4, dadurch gekennzeichnet, daß die Vorratseinrichtungen je eine Wanne (64, 64') und je einen jeweils damit verbundenen Nachfüllbehälter (78 bzw. 78') aufweist, und daß eine Niveauregeleinrichtung vorgesehen ist, um die eine Wanne (64) mit Ergänzungsflüssigkeit aus dem einen Nachfüllbehälter (78) und die andere Wanne (64') mit Verdünnungsflüssigkeit aus dem anderen Nachfüllbehälter (78') nachzufüllen, um die Flüssigkeitspegel in den Wannen (64, 64') innerhalb vorgegebener Grenzen zu halten.

6. Einrichtung nach Anspruch 5, dadurch gekennzeichnet, daß die Nachfüllbehälter (78, 78') geschlossene Behälter sind und oberhalb den Wannen (64 bzw. 64') angeordnet sind, und daß die Niveauregeleinrichtung zwei erste Leitungen (80, 82, 84, 86) und zwei zweite Leitungen (80', 82', 84', 86') aufweist, wobei die ersten Enden der ersten und zweiten Leitungen mit dem Boden des einen (78) oder anderen (78') Nachfüllbehälters verbunden sind und zweite Enden der Leitungen sich in die eine (64) bzw. andere (64') Wanne unterschiedlich weit erstrecken, so daß die Flüssigkeiten in den Wannen (64, 64') auf vorgegebenen Pegeln gehalten werden.

**Claims**

1. Fraction-handling device comprising

— a sample unit having a plurality of probes for taking liquid samples from a corresponding number of measuring vessels of a measuring vessel unit,

— a sample collecting unit having a plurality of pipes for delivering the liquid samples into test tubes arranged in said sample collecting unit below said pipes,

— lines connecting said probes with said pipes, and

— a transport means connected to said lines for transporting the samples from said probes to said test tubes and for transporting replenishing liquid from a replenishing liquid storage means to the probes, characterised in that

— the pipes (56) are provided for both delivering the samples into the test tubes (54) and taking in the replenishing liquid from the storage means (64, 78), for which purpose the pipes (56) can be introduced into the storage means (64, 78),

— the same transport means (6) is provided for transporting the samples from the measuring vessels (16) into the test tubes (54) and for transporting the replenishing from the storage means (64, 78) via the pipes (56) and the probes (30) into the measuring vessels (16), and

— a control means (8) is provided for controlling at least the operation of the sample unit (3), of the sample collecting unit (4) and of the transport means (6).

2. Device according to claim 1, characterised in that the sample collecting unit (4) comprises a first moving means for moving said pipes (56) in an upward and downward direction to a first position in which they are located above the storage means (64, 78) and to a second position in which they are immersed in a replenishing liquid in said storage means (64, 78), and furthermore comprises a second moving means for moving the storage means (64, 78) in a space between the test tubes (54) and the pipes (56) to a position below said pipes to the side of said pipes (56).

3. Device according to claim 1, characterised by

— a further storage means (64', 78') arranged in the sample collecting unit (4) and adapted to contain a dilution liquid, and

— a valve means (92) arranged in said lines (90) in a position between the transport means (6) and the probes (30) and having three valves (92a, 92b, 92c) actuable to allow liquid flow

(a) between the probes (30) and the pipes (56),

(b) between the pipes (56) and a measuring unit (91) connected to one of the valves (92b), and

(c) between the pipes (56) and a container (94) connected to another one of the valves (92c),

— where the pipes (56) are also adapted to be immersed into the dilution liquid in the other storage means (64', 78') and also into the liquid contained in the test tubes (54), and where

— the control means (8) is adapted to control also the operation of the valve means (92).

4. Device according to claim 3, characterised in that the sample collecting unit (4) comprises a first moving means for moving the pipes (56) in an upward and downward direction to a first position in which they are located above the first storage means (64, 78) and the other storage means (64', 78'), to a second position in which they are immersed in the replenishing liquid in the storage means (64, 78) or in the dilution liquid in the other storage means (64', 78'), and to a third position in which they are immersed in the liquid contained in the test tubes (54), and that the sample collecting unit (4) further comprises a second moving means for moving the storage means (64, 78) and the other storage means (64', 78') in the space between the test tubes (54) and the pipes (56) into a position below the pipes (56) and to positions laterally of the pipes (56).

5. Device according to any of claims 1 to 4, characterised in that each of the storage means comprises a storage trough (64, 64') and a storage vessel (78, 78') connected thereto, and that level control means are provided for refilling one storage trough (64) with replenishing liquid from one storage vessel (78) and the other storage trough (64') with dilution liquid from the other storage vessel (78') to keep the liquid levels in the storage troughs (64, 64') within predetermined limits.

6. Device according to claim 5, characterised in that the storage vessels (78, 78') are closed vessels and are mounted above the storage troughs (64 and 64' respectively), and that the level control means comprise two first conduits (80, 82, 84, 86) and two second conduits (80', 82', 84', 86'), wherein the first ends of said first and second conduits are connected to the respective bottoms of the first (78) and second (78') storage vessels, and second ends of the conduits extend into one (64) and into the other (64') storage trough respectively, to different depths whereby the liquid levels in the storage troughs (64, 64') are kept within predetermined limits.

## Revendications

1. Dispositif de traitement de fractions contenant
— un dispositif pour le prélèvement d'échantillons, ayant une pluralité de sondes pour prélever des échantillons liquides d'un nombre correspondent de vases jaugés d'un ensemble de vases jaugés,
— un dispositif-collecteur d'échantillons ayant une pluralité de petits tubes pour délivrer les échantillons liquides dans des récipients d'échantillons disposés dans le dispositif-collecteur d'échantillons au-dessous des petits tubes,
— des conduites reliant les sondes aux petits tubes, et
— un transporteur qui est relié aux conduites pour transporter les échantillons des sondes aux petits tubes et un liquide supplémentaire d'un réservoir de liquide supplémentaire aux sondes, caractérisé par le fait
— que les petits tubes (56) sont destinés non seulement à délivrer les échantillons dans les récipients d'échantillons (54) mais aussi à aspirer le liquide supplémentaire dans le réservoir (64, 78), les petits tubes (56) à cet effet étant introduisables dans la réservoir (64, 78),
— que le même transporteur (6) est destiné à transporter les échantillons des vases jaugés (16) dans les récipients d'échantillons (54) et à transporter le liquide supplémentaire du réservoir (64, 78) par les petits tubes (56) et les sondes (30) dans les vases jaugés (16), et
— qu'un dispositif de commande (8) est prévu pour commander au moins l'opération du dispositif pour le prélèvement d'échantillons (3), du dispositif-collecteur d'échantillons (4) et du transporteur (6).

2. Dispositif selon la revendication 1, caractérisé par le fait que le dispositif-collecteur d'échantillons (4) présente un premier mécanisme-moteur pour mouvoir les petits tubes (56) en haut et en bas dans une première position dans laquelle ils se trouvent au-dessus du réservoir de remplissage (64, 78), et dans une deuxième position dans laquelle ils immergent dans un liquide supplémentaire dans le réservoir de stockage (64, 78), et qu'il présente, en plus, un deuxième mécanisme-moteur pour mouvoir le réservoir de stockage (64, 78) dans un espace entre les récipients d'echantillons (54) et les tubes (56) dans une position au-dessous des petits tubes (56) et dans une position à côté des tubes (56).

3. Dispositif selon la revendication 1, caractérisé par
— un autre dispositif de stockage (64', 78') qui est disposé dans le dispositif-collecteur d'échantillons (4) et qui est capable d'absorber du liquide diluant, et par
— un dispositif-soupape (92) disposé dans les conduites (90) entre le transporteur (6) et les sondes (30), le dispositif-soupape (92) présentant trois soupapes (92a, 92b, 92c) qui peuvent être actionnées de manière à permettre un courant de liquide
(a) entre les sondes (30) et les petits tubes (56),
(b) entre les petits tubes (56) et une mesure (91) qui est raccordée à une des soupapes (92b), et
(c) entre les petits tubes (56) et un vase (94) qui est relié à une autre des soupapes (92c),
— les petits tubes (56) en outre étant disposés de manière qu'ils puissent être immergés dans le liquide diluant contenu dans l'autre réservoir de stockage (64', 78') et également dans le liquide contenu dans le récipient d'échantillons (54), et
— le dispositif de commande étant également destiné à régler le dispositif-soupape (92).

4. Dispositif selon la revendication 3, caractérisé par le fait que le dispositif-collecteur d'échantillons (4) présente un premier dispositif-moteur pour mouvoir les petits tubes (56) en haut et en bas dans une première position dans laquelle ils se trouvent au-dessus du réservoir de stockage (64, 78) et de l'autre réservoir de stockage (64', 78'), dans une deuxième position dans. laquelle ils immergent dans un liquide supplémentaire contenu dans le réservoir de stockage (64, 78) ou dans un liquide diluant contenu dans l'autre réservoir de stockage (64', 78'), et dans une troisième position dans laquelle ils sont immergés dans le liquide contenu dans les récipients d'échantillons (54), et que le dispositif-collecteur d'échantillons (4) présente, en outre, un deuxième dispositif-moteur pour mouvoir le réservoir de stockage (64, 78) et l'autre réservoir de stockage (64', 78') dans l'espace entre les récipients d'échantillons (54) et les petits tubes (56) dans une position au-dessous des petits tubes (56) et dans des positions à côté des petits tubes (56).

5. Dispositif selon l'une quelconque des revendications 1 à 4, caractérisé par le fait que les réservoirs de stockage comprennent chacun une cuve (64, 64') et un réservoir de remplissage (78, 78') relié à celle-ci, et qu'il y a prévu un dispositif-régulateur de niveau pour remplir une cuve (64) avec du liquide supplémentaire contenu dans l'un des réservoirs de remplissage (78) et l'autre cuve (64') avec du liquide diluant contenu dans l'autre des réservoir de remplissage (78') pour maintenir le niveau des liquides dans les cuves (64, 64') au-dedans des limites données.

6. Dispositif selon la revendication 5, caractérisé par le fait que les réservoirs de remplissage (78, 78') sont des réservoirs fermés et qu'ils sont disposés au-dessus des cuves (64, 64'), et que le dispositif-régulateur de niveau présente deux premières conduites (80, 82, 84, 86) et deux deuxièmes conduites (80', 82', 84', 86'), les premiers bouts des premières et des deuxièmes conduites étant reliés au fond de l'un (78) ou de l'autre (78') des réservoirs de remplissage, et les deuxièmes bouts des conduites s'étendant dans l'une (64) ou dans l'autre (64') des cuves à des distances différentes à tel point que les liquides dans les cuves (64, 64') soient maintenus à des niveaux donnés.

# FIG. 1

EP 0 204 343 B1

# FIG. 2

EP 0 204 343 B1

FIG. 3

FIG. 4